# EUROPEAN PATENT SPECIFICATION

(11) **EP 4 598 626 B1**
(45) Date of publication and mention of the grant of the patent: **11.11.2026**
(21) Application number: 23840788.6
(22) Date of filing: 21.12.2023
(51) Int. Cl.: A61N 1/36, A61B 5/026, A61B 5/01, A61B 5/0531, A61N 1/40, A61B 5/28

(54) **POSITIONING ELECTRODE ELEMENTS ON A SUBJECT'S BODY TO PROVIDE STRONGER ALTERNATING ELECTRIC FIELDS (E.G., TTFIELDS) WITHOUT OVERHEATING**
POSITIONIERUNG VON ELEKTRODENELEMENTEN AUF EINEM PATIENTENKÖRPER ZUR BEREITSTELLUNG VON VERSTÄRKTEN ELEKTRISCHEN WECHSELFELDERN (Z.B. TT-FELDER) OHNE ÜBERHITZUNG
POSITIONNEMENT D'ÉLÉMENTS D'ÉLECTRODE SUR LE CORPS D'UN SUJET EN VUE DE LA FOURNITURE DE CHAMPS ÉLECTRIQUES ALTERNATIFS PLUS INTENSES (PAR EXEMPLE, DES CHAMPS TTFIELDS) SANS SURCHAUFFE

(30) Priority: 28.12.2022 US 202263435732 P
(43) Date of publication of application: 13.08.2025
(73) Proprietor: Novocure GmbH, 6340 Baar (CH)
(72) Inventor: WASSERMAN, Yoram, 31905 Haifa (IL); SHTOTLAND, Michael, 31905 Haifa (IL); BEN-TOV KUPERBERG, Mor, 31905 Haifa (IL)
(74) Representative: Carpmaels & Ransford LLP
(86) International application number: PCT/IB2023/063130
(87) International publication number: WO 2024/141897

(56) References cited:
- CN-A- 107 281 635
- US-A1- 2011 264 002
- US-A1- 2016 228 702
- US-A1- 2019 133 673
- US-A1- 2022 313 992

## Description

The present invention relates to a method of determining where to position electrode elements on a subject's body to provide stronger alternating electric fields without overheating.

### BACKGROUND

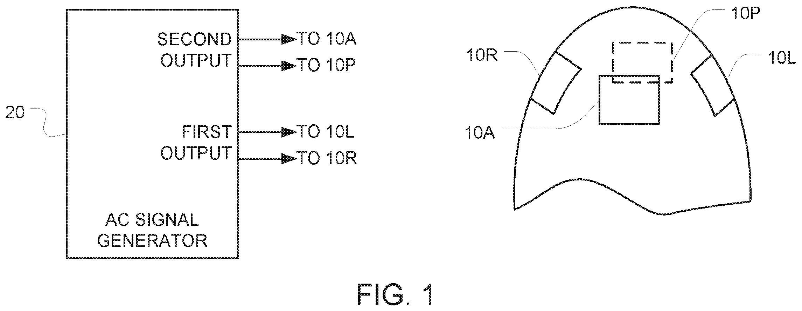

Tumor Treating Fields (TTFields) therapy is a proven approach for treating tumors using alternating electric fields at frequencies between 50 kHz and 1 MHz (e.g., 50 kHz-1 MHz, 50-500 kHz, 75-300 kHz, or 150-250 kHz). FIG. 1 depicts the prior art Optune^{®} system, which delivers TTFields to patients via four transducer arrays 10 that are placed on the patient's skin near the tumor. The transducer arrays 10 are arranged in two pairs, with one pair of transducer arrays 10L, 10R positioned to the left and right of the tumor, and the other pair of transducer arrays 10A, 10P positioned anterior and posterior to the tumor. Each transducer array is connected via a multi-wire cable to an AC signal generator 20. The AC signal generator (a) sends an AC current through the anterior/posterior (A/P) pair of transducer arrays for 1 second, which induces an electric field with a first direction through the tumor; then (b) sends an AC current through the left/right (L/R) pair of arrays for 1 second, which induces an electric field with a second direction through the tumor; then repeats steps (a) and (b) for the duration of the treatment. Each transducer array includes a plurality (e.g., between 9 and 30) of electrode elements.

Alternating electric fields can also be used to treat medical conditions other than tumors. For example, as described in U.S. Patent No. 10,967,167, alternating electric fields can be used to increase the permeability of the blood brain barrier so that, e.g., chemotherapy drugs can reach the brain.

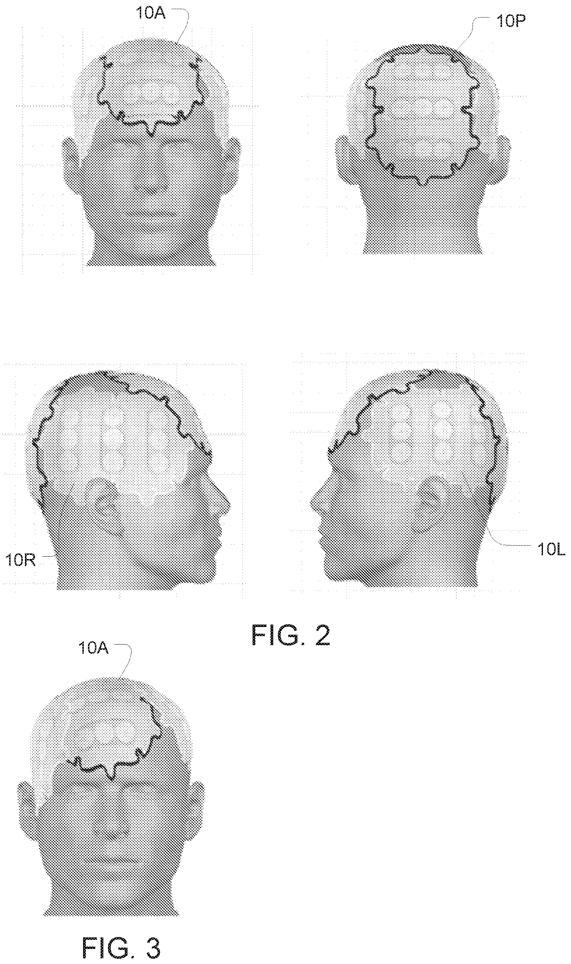

FIG. 2 depicts a more detailed view of a set of four transducer arrays 10 in which the individual electrode elements that make up each of the transducer arrays are visible. In this example, each of the transducer arrays 10 includes nine round electrode elements that are supported by a self-adhesive substrate. Notably, although FIG. 2 depicts each of the four transducer arrays 10 positioned at a particular place on a patient's head, the exact positioning of each of the transducer arrays 10 during use can vary with respect to the position depicted in FIG. 2 by a few centimeters in the up, down, right, left, front, and/or back directions. In addition, one or more of the transducer arrays 10 can be rotated (e.g., by 0-30° with respect to the position depicted in FIG. 2) to a different position (e.g., the position depicted in FIG. 3 for the anterior transducer array 10A).

Increasing the strength of alternating electric fields will typically increase the efficacy of treatment. Existing software (e.g., Novotal^{™}) simulates the strength of the TTFields within the subject's head to recommend exactly where to position each of the transducer arrays 10 on the subject's head for a particular patient. This software operates by using electrical characteristics of voxels within the subject's head to calculate what the field strength will be in the relevant target region (e.g., in a tumor) when different transducer array positionings are used to apply TTFields. The software then recommends a transducer positioning that will maximize the field strength in the target region. Notably, while accepting the recommended positions from the field-simulation software will almost always improve the strength of TTFields that reach the target region, in many situations the field strength will only improve by a small amount (e.g., about 5-10%).

CN-A-107281635 discloses an electrode physiotherapeutic device comprising one or more pairs of electrodes, a control device and an electrode-positioning device, and the electrode-positioning device is configured to receive or establish a three-dimensional model of the brain of the user and determine attachment positions of the electrodes based on the three-dimensional model and the target region.

### SUMMARY OF THE INVENTION

The invention is directed to a method of determining where to position a plurality of electrode elements on a subject's body according to claim 1.

Embodiments of the invention are disclosed in the dependent claims.

### BRIEF DESCRIPTION OF THE DRAWINGS

FIG. 1 is a block diagram of the prior art Optune^{®} system for delivering TTFields.
FIG. 2 depicts a set of transducer arrays that may be used for delivering TTFields, positioned at respective positions on a subject's head.
FIG. 3 depicts the same set of transducer arrays, but positioned at respective different positions on a subject's head.
FIG. 4 depicts a schematic representation of a transducer array for delivering TTFields to a subject's body, and a schematic representation of a thermal image of a portion of the subject's body upon which the transducer array can be positioned.
FIG. 5 is a histogram that shows the coolest 20% and the warmest 70% of the total area for an exemplary data set.
FIG. 6 depicts three possible positionings of the FIG. 4 transducer array, each of which shows where each of the electrode elements E1-E9 lies on the portion 50 for each of three positions A, B, C.

Various embodiments are described in detail below with reference to the accompanying drawings, wherein like reference numerals represent like elements.

### DESCRIPTION OF THE PREFERRED EMBODIMENTS

This application describes a new approach for determining where to position each of the transducer arrays on the subject's head (or other body part) for a given subject. This new approach relies on local variations in the ability of the given subject's body to carry heat away from the electrode elements on the transducer arrays.

When applying TTFields to a subject's body (e.g., using Optune^{®}), the amplitude of the AC current that can be applied to a given subject's body is usually limited by thermal considerations. More specifically, because the Optune^{®} transducer arrays heat up when the amplitude of the AC current that is applied to those transducers increases, a safety temperature threshold (e.g., 39° C) is eventually reached. And that temperature threshold has traditionally been the limiting factor that prevented Optune^{®} from operating at higher amplitudes.

The inventors have recognized that some regions on the surface of a given subject's body are significantly better at carrying heat away (i.e., sinking heat) from the transducer arrays' electrode elements, as compared to other regions that may only be a short distance (e.g., 5 cm) away. And these differences between the heat-sinking ability of different regions of the subject's body can account for a dramatic increase in the amplitude of the AC current that can be applied to a given subject's body. This is because the electrode elements that are positioned over regions that are more effective at carrying heat away can carry higher currents without reaching the safety temperature threshold.

In view of this phenomena, it becomes possible to increase the amplitude of TTFields that can be delivered to a given subject by shifting the position of the transducer arrays so that all (or at least most) of the electrode elements are positioned over regions that are more effective at carrying heat away, and so that very few (or none) of the electrodes are positioned over regions that are less effective at carrying heat away. And notably, when the electrode elements are positioned as described in the previous sentence, the heat sinking provided by the subject's body can facilitate an increase in current on the order of 20% without overheating (as compared to when the electrode elements are positioned over regions that are less effective at carrying heat away).

Because the ~20% increase in current provided by this approach based on heat-sinking can exceed the increase in current that is provided by the prior art approach based on field-strength simulation, using the former approach can provide better results than the latter approach. This is because the 20% increase in field strength provided by the former approach outweighs the 5-10% loss in the field strength that flows from placing the transducer arrays at a sub-optimal position (from the perspective of field-strength simulations).

Optionally, the improvement provided by the positioning recommendations that are based on the heat-sinking characteristics of different regions of the subject's skin can be compared to the improvement provided by the prior art field-simulating software. When the increase in current provided by positioning the transducer arrays based on the heat-sinking characteristics of different regions of the subject's skin exceeds the increase in field strength provided by the prior art field-simulating software, the former approach should be implemented. On the other hand, when the increase in current provided by positioning the transducer arrays based on the heat-sinking characteristics of different regions of the subject's skin is less than the increase in field strength provided by the prior art field-simulating software, the latter approach should be implemented. Alternatively, a hybrid approach that considers both the heat-sinking characteristics and field simulations can be implemented.

Without being bound by this theory, the inventors believe that one factor that impacts various regions' abilities to carry heat away from the electrode elements is the differences in the amount of blood that flows adjacent to the surface of those regions (i.e., within a few millimeters of the surface of those regions). Regions with higher blood flow are more effective in carrying heat away from electrode elements placed on those regions. Accordingly, ascertaining which regions have relatively high blood flows adjacent to the subject's skin can also identify the regions that are more effective at carrying heat away.

One suitable approach to identify such regions relies on thermal imaging to ascertain which regions of a subject's body have relatively high blood flows adjacent to the subject's skin. The first step in this approach is positioning the subject in an environment with an ambient temperature that is below the subject's body temperature (e.g., in a room that is below 35° C, below 30° C, below 25° C, below 22° C, or below 20° C). Then, a thermal image of the portion of the subject's body where the transducer arrays will be positioned is obtained (e.g., using an infrared camera). Because the subject's blood is warmer than the ambient temperature, regions of the subject's body that have higher blood flows will be warmer in the thermal image, and regions of the subject's body that have lower blood flows will be cooler in the thermal image. Positions for the electrode elements are then selected based at least in part on the thermal image (e.g., by positioning most or all of the electrode elements on warmer regions, and very few (or none) of the electrode elements on cooler regions).

Regions of a subject's body that have relatively high blood flows adjacent to the subject's skin can also be detected using thermal imaging in the opposite direction. In this example, the subject is positioned in an environment with an ambient temperature that is significantly *higher* than the subject's body temperature (e.g., in a room, sauna, etc. that is hotter than 40° C, hotter than 45° C, hotter than 50° C, hotter than 55° C, or hotter than 60° C, or beneath a heating blanket). Then, a thermal image of the portion of the subject's body where the transducer arrays will be positioned is obtained (e.g., using an infrared camera). Because the subject's blood is cooler than the ambient temperature, regions of the subject's body that have higher blood flows will be cooler in the thermal image, and regions of the subject's body that have lower blood flows will be hotter in the thermal image. Positions for the electrode elements are then selected based at least in part on the thermal image (e.g., by positioning most or all of the electrode elements on cooler regions, and very few (or none) of the electrode elements on warmer regions).

Another suitable approach relies on impedance tomography to ascertain which regions of a subject's body may have relatively better heat-sinking abilities adjacent to the subject's skin with respect to the heat generated in or near the electrodes during TTFields treatment. This approach uses impedance tomography to determine the impedance of voxels located adjacent to the surface of the subject's body (e.g., < 1 mm, < 2 mm, < 3 mm, < 4 mm, or < 5 mm of depth into the subject's body, such as from 0.5 to 5 mm, or from 1 to 5 mm, or from 2 to 5 mm, or from 3 to 5 mm, or from 4 to 5 mm from the surface of the subject's body). Notably, the impedance measurements need not be performed while TTFields treatment is occurring. Any conventional approach for performing the impedance tomography may be used. Regions of the subject's body that present low impedance pathways close to the surface of the body have lower impedances (and higher conductances), and better heat-sinking abilities with respect to the heat from the TTFields treatment. Positions for the electrode elements are then selected based at least in part on the impedances (or conductances) adjacent to the surface of the subject's body (e.g., by positioning most or all of the electrode elements on low impedance regions, and very few (or none) of the electrode elements on higher impedance regions).

Yet another suitable approach relies on an array of electrode elements to ascertain which regions of a subject's body may have relatively better heat-sinking abilities adjacent to the subject's skin with respect to the heat generated in or near the electrodes during TTFields treatment. This approach uses a set of electrodes to measure the surface impedance (or surface conductance) of the subject's body. In these embodiments, an array of electrode elements is positioned on the subject's body at the location where the transducer arrays will be placed. The electrodes can be, for example, standard electrocardiogram electrodes or the like, optionally affixed to a flexible substrate that is configured to conform to a particular body part. Currents and/or voltages between respective pairs of the electrode elements within the arrays are then measured, and a map of surface impedance (or surface conductance) is generated based on the measured currents and/or voltages. Here again, regions of the subject body that present low impedance pathways close to the surface of the body have lower impedances (and higher conductances), and better heat-sinking abilities with respect to the heat from the TTFields treatment. Positions for the electrode elements are then selected based at least in part on the impedances (or conductances) adjacent to the surface of the subject's body (e.g., by positioning most or all of the electrode elements on low impedance regions, and very few (or none) of the electrode elements on higher impedance regions).

Any of these approaches can be used to determine where to position a plurality of electrode elements on a subject's body so that higher currents can be driven through the electrode elements during a TTFields treatment session without overheating the electrode elements.

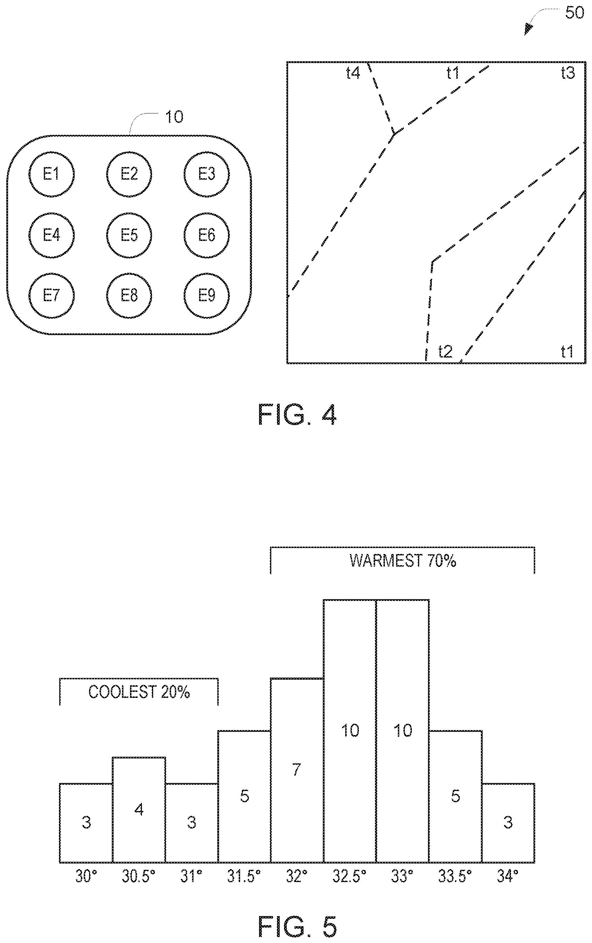

FIGS. 4-6 depict an example of how the first of these approaches (i.e., the thermal imaging approach) can be used to ascertain which regions of a subject's body have relatively high blood flows adjacent to the subject's skin, and how this information can be used to determine where to position the electrode elements on the subject's body so that higher currents can be driven through the electrode elements.

The left half of FIG. 4 depicts an example of a transducer array 10 in which the electrode elements E1-E9 are arranged in a particular pattern. And the right half of FIG. 4 is an example schematic representation of a thermal image of a portion 50 of the subject's body upon which the plurality of electrode elements can be positioned in connection with a treatment session (e.g., a thermal image of the left side of a subject's head that is about to be treated using TTFields). In this example, the regions t1 are the coolest regions in the thermal image, region t2 is warmer than region t1, region t3 is warmer than region t2, and region t4 is warmer than region t3 (which makes region t4 the warmest region).

Based on the relative temperatures of the regions t1-t4, we conclude that the regions t1 have the lowest blood flow, the blood flow in region t2 is higher than in region t1, the blood flow in region t3 is higher than in region t2, and region t4 has the highest blood flow.

In this example (which is not drawn to scale), the two t1 regions collectively occupy 20% of the total area of the portion 50, the t2 region occupies 10% of the total area, the t3 region occupies 50% of the total area, and the t4 region occupies 20% of the total area. This means that region t1 corresponds to the coolest 20% of the total area, and that regions t3 and t4 collectively correspond to the warmest 70% of the total area.

FIG. 5 is an example of a histogram to clarify what is meant by the coolest 20% of the total area and the warmest 70% of the total area. This histogram depicts an example in which the portion of the subject's body upon which the plurality of electrode elements will be positioned was divided into 50 equal regions, and the temperature of each of those regions was measured. In this example, three of the regions had a temperature of 30° C, four of the regions had a temperature of 30.5° C, three of the regions had a temperature of 31° C, five of the regions had a temperature of 31.5° C, seven of the regions had a temperature of 32° C, ten of the regions had a temperature of 32.5° C, ten of the regions had a temperature of 33° C, five of the regions had a temperature of 33.5° C, and three of the regions had a temperature of 34°C. Thus in this example, the 10 regions that are at or below 31° C correspond to the coolest 20% of the total area, and the 35 regions that are at or above 32° C correspond to the warmest 70% of the total area.

Because regions with higher blood flow are more efficient at carrying heat away from the electrode elements on the transducer array, it is best to position as many electrode elements as possible on the regions with higher blood flows, and as few elements as possible on the regions with lower blood flows. One example of how to achieve these two goals is to position the transducer arrays at a position that maximizes an average temperature of all regions of the thermal image that underlie the plurality of electrode elements.

Another example of how to achieve these two goals is to position the transducer arrays in accordance with the following two guidelines: (a) most of the electrode elements should overlie portions of the subject's body that correspond to the warmest 70% of the thermal image (i.e., regions t3 and t4 in FIG. 4); and (b) none of the electrode elements should overlie portions of the subject's body that correspond to the coolest 20% of the thermal image (i.e., region t1 in FIG. 4). Although it would be ideal to select a layout that conforms with both guidelines (a) and (b), in certain circumstances this may not be possible (depending on the layout of the thermal image). In these circumstances, following only one of the guidelines will suffice.

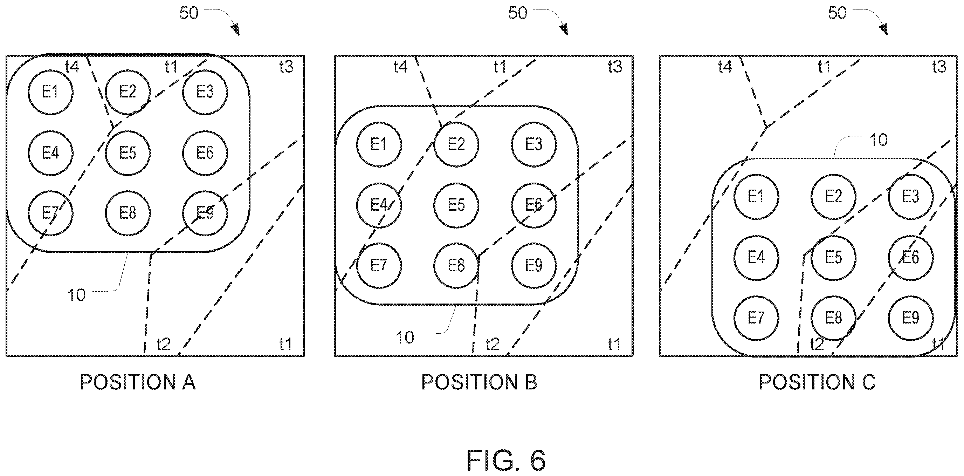

The transducer array 10 depicted in FIG. 4 can be positioned anywhere on the portion 50, and FIG. 6 depicts three possible positionings of the transducer array on the portion 50. We shall now examine where each of the electrode elements E1-E9 lies for each of the three positions A, B, C depicted in FIG. 6, in view of guidelines (a) and (b).

If the transducer array 10 is affixed to the subject's body at position A, most of the electrode elements E1-E9 will overlie regions T3 and T4, which satisfies guideline (a). But guideline (b) is not satisfied because electrode element E2 overlies region t1 (which corresponds to the coolest 20% of the thermal image).

If the transducer array 10 is affixed to the subject's body at position B, most of the electrode elements E1-E9 will overlie regions t3 and t4, which satisfies guideline (a). And none of the electrode elements overlie region t1, which satisfies guideline (b).

Finally, if the transducer array 10 is affixed to the subject's body at position C, electrode elements E3, E5, E6, E8, and E9 are not located in regions t3/t4, which means that guideline (a) is not satisfied. And guideline (b) is also not satisfied because electrode element E9 is located in region t1 (which corresponds to the coolest 20% of the thermal image).

Based on the previous few paragraphs, we see that the only position that satisfies both guideline (a) and guideline (b) is position B. We can therefore conclude that position B is superior to positions A and C. After this conclusion is reached, the transducer array is placed on the subject's body at whichever position has been found to be superior. The electrode elements will therefore lie over the selected positions.

Another approach is to position as many portions of the electrode elements as possible on the regions with higher blood flows, and/or as few portions of the electrode elements as possible on the regions with lower blood flows. This can be accomplished by selecting positions at which most portions of the electrode elements (i.e., >50% of the total collective area of the electrode elements) overlie portions of the subject's body that correspond to the warmest 70% of the thermal image or selecting positions at which no portions of the electrode elements overlie portions of the subject's body that correspond to the coolest 20% of the thermal image. Yet another approach is to select positions at which (a) most portions of the electrode elements overlie portions of the subject's body that correspond to the warmest 70% of the thermal image and (b) no portions of the electrode elements overlie portions of the subject's body that correspond to the coolest 20% of the thermal image.

After the transducer array has been placed on the subject's body, the transducer arrays can be used to apply an alternating electric field at a frequency between 50 kHz and 1 MHz (e.g., 50 kHz-1 MHz, 50-500 kHz, 75-300 kHz, or 150-250 kHz) to the subject's body. This may be accomplished by applying an AC current to transducer arrays positioned on opposite sides of the target region at a corresponding frequency.

Note that the numeric values in guidelines (a) and (b) above are only examples, and those numeric values can be varied (e.g., based on the nature of the thermal image for a given patient). For example, for guideline (a), the warmest 70% of the thermal image could be replaced with the warmest 50%, 65%, 75%, or 80%. And for guideline (b), the coolest 20% of the thermal image could be replaced with the coolest 5%, 10%, 15%, or 25%.

Of course, if the subject is positioned in an environment with an ambient temperature that is significantly *higher* than the subject's body temperature (such as a temperature that is hotter than 40° C, hotter than 45° C, hotter than 50° C, hotter than 55° C, or hotter than 60° C, or beneath a heating blanket), the role of the warmest and coolest regions will be reversed. In this case, the transducer arrays can be positioned at locations that minimize an average temperature of all regions of the thermal image that underlie the plurality of electrode elements. Alternatively, the transducer arrays could be positioned so that (i) most of the electrode elements should overlie portions of the subject's body that correspond to the coolest 70% of the thermal image; and (ii) none of the electrode elements should overlie portions of the subject's body that correspond to the warmest 20% of the thermal image. Although it would be ideal to select a layout that satisfies both conditions (i) and (ii), in certain circumstances this may not be possible (depending on the layout of the thermal image). In these circumstances, satisfying only one of those conditions can suffice.

Another approach is to position as many portions of the electrode elements as possible on the regions with higher blood flows, and/or as few portions of the electrode elements as possible on the regions with lower blood flows. In the "hot room" embodiments, this can be accomplished by selecting positions at which most portions of the electrode elements (i.e., >50% of the total collective area of the electrode elements) overlie portions of the subject's body that correspond to the coolest 70% of the thermal image or selecting positions at which no portions of the electrode elements overlie portions of the subject's body that correspond to the warmest 20% of the thermal image. Yet another approach is to select positions at which (a) most portions of the electrode elements overlie portions of the subject's body that correspond to the coolest 70% of the thermal image and (b) no portions of the electrode elements overlie portions of the subject's body that correspond to the warmest 20% of the thermal image.

Note once again that the numeric values in this example are only examples, and those numeric values can be varied (e.g., based on the nature of the thermal image for a given patient). For example, for condition (i), the coolest 70% of the thermal image could be replaced with the coolest 50%, 65%, 75%, or 80%. And for condition (ii), the warmest 20% of the thermal image could be replaced with the warmest 5%, 10%, 15%, or 25%.

FIGS. 4-6 explain how the thermal imaging approach can be used to ascertain which regions of a subject's body have relatively high blood flows adjacent to the subject's skin, and how this information can be used to determine where to position the electrode elements on the subject's body so that higher currents can be driven through the electrode elements. But any of the other approaches described herein for ascertaining which regions of a subject's body have enhanced heat-sinking abilities adjacent to the subject's skin may be used.

In one example, an approach that is based on impedance tomography is used. In this approach, instead of generating a thermal image as described above in connection with FIGS. 4-6 and using the temperature for each region as a surrogate for blood flow, a map of the surface impedance is generated using impedance tomography, and the impedance of each region is used as a surrogate for heat-sinking abilities (with lower impedances corresponding to higher heat-sinking abilities).

In another example, an approach that is based on impedance measurement using an array of electrodes (e.g., electrodes that are similar to ECG electrodes) is used. In this approach, instead of generating a thermal image as described above in connection with FIGS. 4-6 and using the temperature or each region as a surrogate for blood flow, a map of the surface impedance is generated by applying currents and/or voltages to the array of electrodes to measure the impedance of each region, and the impedance of each region is used as a surrogate for heat-sinking ability (with lower impedances corresponding to higher heat-sinking abilities).

As explained above, regions with higher heat-sinking abilities are more efficient at carrying heat away from the electrode elements on the transducer array, so it is best to position as many electrode elements as possible on the regions with higher heat-sinking abilities, and as few elements as possible on the regions with lower heat-sinking abilities. When either of these impedance-based approaches are used, these two goals can be achieved by positioning the transducer arrays at positions that minimizes an average impedance of all regions of the impedance map that underlie the plurality of electrode elements.

These two goals can also be achieved by positioning the transducer arrays in accordance with the following two guidelines: (a) most of the electrode elements should overlie portions of the subject's body that correspond to the lowest-impedance 70% of the impedance map; and (b) none of the electrode elements should overlie portions of the subject's body that correspond to the highest-impedance 20% of the impedance map. Although it would be ideal to select a layout that conforms with both guidelines (a) and (b), in certain circumstances this may not be possible (depending on the layout of the impedance map). In these circumstances, following only one of the guidelines will suffice. Another approach is to select positions at which most portions of the electrode elements (i.e., >50% of the total collective area of the electrode elements) overlie portions of the subject's body that correspond to the lowest-impedance 70% of the impedance map or selecting positions at which no portions of the electrode elements overlie portions of the subject's body that correspond to the highest-impedance 20% of the impedance map. Yet another approach is to select positions at which (a) most portions of the electrode elements overlie portions of the subject's body that correspond to the lowest-impedance 70% of the impedance map and (b) no portions of the electrode elements overlie portions of the subject's body that correspond to the highest-impedance 20% of the impedance map.

Note that the numeric values in guidelines (a) and (b) above are only examples, and those numeric values can be varied (e.g., based on the nature of the impedance map for a given patient). For example, for guideline (a), the lowest-impedance 70% of the impedance map could be replaced with the lowest-impedance 50%, 65%, 75%, or 80%. And for guideline (b), the highest-impedance 20% of the impedance map could be replaced with the highest-impedance 5%, 10%, 15%, or 25%.

In some embodiments, data obtained using the thermal-imaging approach described above can be combined with data obtained using the impedance-or-conductance measuring approach described above to determine where the transducer arrays should be positioned.

After a conclusion of where to position the transducer array is reached, the transducer array is placed on the subject's body at whichever position has been found to be superior based on the impedance map. The electrode elements will therefore lie over the selected positions. After the transducer array has been placed on the subject's body, the transducer arrays can be used to apply an alternating electric field at a frequency between 50 kHz and 1 MHz (e.g., 50 kHz-1 MHz, 50-500 kHz, 75-300 kHz, or 150-250 kHz) to the subject's body. This may be accomplished by applying an AC current to transducer arrays positioned on opposite sides of the target region at a corresponding frequency.

Headings are provided for convenience only and are not to be construed to limit the invention in any manner. Embodiments illustrated under any heading or in any portion of the disclosure may be combined with embodiments illustrated under the same or any other heading or other portion of the disclosure. Any combination of the elements described herein in all possible variations thereof is encompassed by the invention unless otherwise indicated herein or otherwise clearly contradicted by context.

While the present invention has been disclosed with reference to certain embodiments, numerous modifications, alterations, and changes to the described embodiments are possible without departing from the scope of the present invention, as defined in the appended claims. Accordingly, it is intended that the present invention not be limited to the described embodiments, but that it has the full scope defined by the language of the following claims.

## Claims

1. A method of determining where to position a plurality of electrode elements (E1-E9) on a subject's body so that higher currents can be driven through the electrode elements (E1-E9) during a treatment session without overheating the electrode elements (E1-E9), the method comprising:
mapping at least one heat-sinking characteristic of a portion (50) of the subject's body upon which the electrode elements (E1-E9) can be positioned in connection with the treatment session; and
selecting positions for the electrode elements (E1-E9) based at least in part on the at least one mapped heat-sinking characteristic.

2. The method of claim 1, wherein the mapping comprises thermal imaging of the portion (50) of the subject's body.

3. The method of claim 2, wherein the thermal imaging is performed using an infrared camera.

4. The method of claim 2, wherein the mapping is performed while the portion (50) of the subject's body is located in an environment with an ambient temperature below 25° C.

5. The method of claim 2, wherein the mapping is performed while the portion (50) of the subject's body is located in an environment with an ambient temperature above 45° C.

6. The method of claim 2, wherein the selecting comprises selecting positions at which most portions of the electrode elements (E1-E9) overlie portions of the subject's body that correspond to the warmest 70% of the thermal image.

7. The method of claim 2, wherein the selecting comprises selecting positions at which no portions of the electrode elements (E1-E9) overlie portions of the subject's body that correspond to the coolest 20% of the thermal image.

8. The method of claim 2, wherein the selecting comprises selecting positions that maximize an average temperature of all regions of the thermal image that underlie the electrode elements (E1-E9).

9. The method of claim 1, wherein the mapping comprises measuring surface impedance or surface conductance of the portion (50) of the subject's body.

10. The method of claim 9, wherein the mapping comprises making impedance or conductance measurements within 5 mm of a surface of skin of the subject's body.

11. The method of claim 9, wherein the selecting comprises selecting positions that minimize an average impedance of all regions of the subject's body that underlie the electrode elements (E1-E9).

12. The method of claim 9, wherein the selecting comprises selecting positions at which most of the electrode elements (E1-E9) overlie portions of the subject's body that correspond to the lowest 70% of the impedances of the mapping or the highest 70% of the conductances of the mapping.

13. The method of claim 9, wherein the selecting comprises selecting positions at which none of the electrode elements (E1-E9) overlie portions of the subject's body that correspond to the highest 20% of the impedances of the mapping or the lowest 20% of the conductances of the mapping.

14. The method of claim 1, wherein the selecting of positions for the electrode elements (E1-E9) is also based on a plurality of field strength simulations.

15. The method of claim 1, wherein the mapping comprises thermal imaging of the portion (50) of the subject's body and measuring surface impedance or surface conductance of the portion (50) of the subject's body, and the selecting is based at least in part on data from the thermal imaging and the surface impedance or surface conductance measurement.

## Patentansprüche

1. Verfahren zum Bestimmen, wo eine Vielzahl von Elektrodenelementen (E1-E9) auf dem Körper eines Subjekts positioniert werden soll, so dass während einer Behandlungssitzung höhere Ströme durch die Elektrodenelemente (E1-E9) getrieben werden kann, ohne die Elektrodenelemente (E1-E9) zu überhitzen, wobei das Verfahren Folgendes umfasst:
Kartieren mindestens einer Wärmeableitungscharakteristik eines Abschnitts (50) des Körpers des Subjekts, auf dem die Elektrodenelemente (E1-E9) in Verbindung mit der Behandlungssitzung positioniert werden können; und
Auswähle von Positionen für die Elektrodenelemente (E1-E9) zumindest teilweise auf Grundlage der mindestens einen kartierten Wärmeableitungscharakteristik.

2. Verfahren nach Anspruch 1, wobei das Kartieren eine Wärmebildgebung des Abschnitts (50) des Körpers des Subjekts umfasst.

3. Verfahren nach Anspruch 2, wobei die Wärmebildgebung unter Verwendung einer Infrarotkamera durchgeführt wird.

4. Verfahren nach Anspruch 2, wobei das Kartieren durchgeführt wird, während sich der Abschnitt (50) des Körpers des Subjekts in einer Umgebung mit einer Umgebungstemperatur unter 25° C befindet.

5. Verfahren nach Anspruch 2, wobei das Kartieren durchgeführt wird, während sich der Abschnitt (50) des Körpers des Subjekts in einer Umgebung mit einer Umgebungstemperatur über 45° C befindet.

6. Verfahren nach Anspruch 2, wobei das Auswählen das Auswählen von Positionen umfasst, an denen die meisten Abschnitte der Elektrodenelemente (E1-E9) über Abschnitten des Körpers des Subjekts liegen, die den wärmsten 70 % des Wärmebildes entsprechen.

7. Verfahren nach Anspruch 2, wobei das Auswählen das Auswählen von Positionen umfasst, an denen keine Abschnitte der Elektrodenelemente (E1-E9) über Abschnitten des Körpers des Subjekts liegen, die den kühlsten 20 % des Wärmebildes entsprechen.

8. Verfahren nach Anspruch 2, wobei das Auswählen das Auswählen von Positionen umfasst, die eine durchschnittliche Temperatur aller Bereiche des Wärmebildes maximieren, die unter den Elektrodenelementen (E1-E9) liegen.

9. Verfahren nach Anspruch 1, wobei das Kartieren die Messung der Oberflächenimpedanz oder der Oberflächenleitfähigkeit des Abschnitts (50) des Körpers des Subjekts umfasst.

10. Verfahren nach Anspruch 9, wobei das Kartieren das Vornehmen von Impedanz- oder Leitwertmessungen innerhalb von 5 mm einer Hautoberfläche des Körpers des Subjekts umfasst.

11. Verfahren nach Anspruch 9, wobei das Auswählen das Auswählen von Positionen umfasst, die eine durchschnittliche Impedanz aller Bereiche des Körpers des Subjekts minimieren, die unter den Elektrodenelementen (E1-E9) liegen.

12. Verfahren nach Anspruch 9, wobei das Auswählen das Auswählen von Positionen umfasst, an denen die meisten Elektrodenelemente (E1-E9) über Abschnitten des Körpers des Subjekts liegen, die den niedrigsten 70 % der Impedanzen der Kartierung oder den höchsten 70 % der Leitwerte der Kartierung entsprechen.

13. Verfahren nach Anspruch 9, wobei das Auswählen das Auswählen von Positionen umfasst, an denen keines der Elektrodenelemente (E1-E9) über Abschnitten des Körpers des Subjekts liegt, die den höchsten 20 % der Impedanzen der Kartierung oder den niedrigsten 20 % der Leitwerte der Kartierung entsprechen.

14. Verfahren nach Anspruch 1, wobei das Auswählen von Positionen für die Elektrodenelemente (E1-E9) ebenfalls auf einer Vielzahl von Feldstärkesimulationen beruht.

15. Verfahren nach Anspruch 1, wobei das Kartieren eine Wärmebildgebung des Abschnitts (50) des Körpers des Subjekts und das Messen der Oberflächenimpedanz oder des Oberflächenleitwerts des Abschnitts (50) des Körpers des Subjekts umfasst und das Auswählen zumindest teilweise auf Daten aus der Wärmebildgebung und der Oberflächenimpedanz- oder Oberflächenleitwertmessung beruht.

## Revendications

1. Procédé permettant de déterminer l'endroit où positionner une pluralité d'éléments d'électrode (E1-E9) sur le corps d'un sujet afin de faire passer des courants plus élevés à travers les éléments d'électrode (E1-E9) pendant une séance de traitement sans surchauffer les éléments d'électrode (E1-E19), le procédé comprenant :
la cartographie d'au moins une caractéristique de dissipation thermique d'une partie (50) du corps du sujet sur laquelle les éléments d'électrode (E1-E9) peuvent être positionnés dans le cadre de la séance de traitement ; et
la sélection des positions des éléments d'électrode (E1-E9) sur la base au moins en partie de la au moins une caractéristique de dissipation thermique cartographiée.

2. Procédé selon la revendication 1, dans lequel la cartographie comprend l'imagerie thermique de la partie (50) du corps du sujet.

3. Procédé selon la revendication 2, dans lequel l'imagerie thermique est réalisée à l'aide d'une caméra infrarouge.

4. Procédé selon la revendication 2, dans lequel la cartographie est réalisée lors de la localisation de la partie (50) du corps du sujet dans un environnement dont la température ambiante est inférieure à 25 °C.

5. Procédé selon la revendication 2, dans lequel la cartographie est réalisée lors de la localisation de la partie (50) du corps du sujet dans un environnement dont la température ambiante est supérieure à 45 °C.

6. Procédé selon la revendication 2, dans lequel la sélection comprend la sélection de positions auxquelles la plupart des parties des éléments d'électrode (E1-E9) recouvrent des parties du corps du sujet qui correspondent aux 70 % les plus chauds de l'image thermique.

7. Procédé selon la revendication 2, dans lequel la sélection comprend la sélection de positions auxquelles aucune partie des éléments d'électrode (E1-E9) ne recouvre les parties du corps du sujet qui correspondent aux 20 % les plus froids de l'image thermique.

8. Procédé selon la revendication 2, dans lequel la sélection comprend la sélection de positions qui maximisent la température moyenne de toutes les régions de l'image thermique qui sous-tendent les éléments d'électrode (E1-E9).

9. Procédé selon la revendication 1, dans lequel la cartographie comprend la mesure de l'impédance de surface ou de la conductance de surface de la partie (50) du corps du sujet.

10. Procédé selon la revendication 9, dans lequel la cartographie comprend la réalisation de mesures d'impédance ou de conductance à moins de 5 mm d'une surface de peau du corps du sujet.

11. Procédé selon la revendication 9, dans lequel la sélection comprend la sélection de positions qui minimisent l'impédance moyenne de toutes les régions du corps du sujet qui sous-tendent les éléments d'électrode (E1-E9).

12. Procédé selon la revendication 9, dans lequel la sélection comprend la sélection de positions auxquelles la plupart des éléments d'électrode (E1-E9) recouvrent des parties du corps du sujet qui correspondent aux 70 % les plus faibles des impédances de la cartographie ou aux 70 % les plus élevées des conductances de la cartographie.

13. Procédé selon la revendication 9, dans lequel la sélection comprend la sélection de positions auxquelles aucun des éléments d'électrode (E1-E9) ne recouvre des parties du corps du sujet qui correspondent aux 20 % les plus élevées des impédances de la cartographie ou aux 20 % les plus faibles des conductances de la cartographie.

14. Procédé selon la revendication 1, dans lequel la sélection de positions des éléments d'électrode (E1-E9) est également basée sur une pluralité de simulations d'intensité de champ.

15. Procédé selon la revendication 1, dans lequel la cartographie comprend l'imagerie thermique de la partie (50) du corps du sujet et la mesure de l'impédance de surface ou de la conductance de surface de la partie (50) du corps du sujet, et la sélection est basée au moins en partie sur les données de l'imagerie thermique et de la mesure de l'impédance de surface ou de la conductance de surface.
